# EUROPEAN PATENT SPECIFICATION

(11) **EP 4 132 498 B1**
(45) Date of publication and mention of the grant of the patent: **14.10.2026**
(21) Application number: 21784811.8
(22) Date of filing: 09.04.2021
(51) Int. Cl.: A61K 31/138, A61K 9/28, A61P 25/18

(54) **ENDOXIFEN CITRATE DOSAGE REGIMEN FOR THE TREATMENT OF BIPOLAR I DISORDER**
ENDOXIFENCITRAT-DOSIERUNGSSCHEMA ZUR BEHANDLUNG DER BIPOLAREN STÖRUNG TYP I
POSOLOGIE DU CITRATE D'ENDOXIFÈNE POUR LE TRAITEMENT DU TROUBLE BIPOLAIRE DE TYPE I

(30) Priority: 10.04.2020 US 202063008162 P
(43) Date of publication of application: 15.02.2023
(73) Proprietor: Jina Pharmaceuticals, Inc., Libertyville, IL 60048 (US)
(72) Inventor: AHMAD, Ateeq, Wadsworth, Illinois 60083 (US); AHMAD, Imran, Libertyville, Illinois 60048 (US); AHMAD, Moghisuddin, Wadsworth, Illinois 60083 (US); ALI, Shoukath M, Vernon Hills, Illinois 60061 (US); SHEIKH, Saifuddin, Libertyville, Illinois 60048 (US)
(74) Representative: HGF
(86) International application number: PCT/IB2021/052972
(87) International publication number: WO 2021/205404

(56) References cited:
- WO-A1-2008/048194
- WO-A1-2021/003433
- WO-A2-2009/152430
- US-A1- 2012 201 908
- US-A1- 2019 231 687
- AHMAD ATEEQ ET AL: "Endoxifen: A new, protein kinase C inhibitor to treat acute and mixed mania associated with bipolar I disorder", BIPOLAR DISORDERS, vol. 23, no. 6, 25 December 2020 (2020-12-25), DK, pages 595 - 603, XP093129590, ISSN: 1398-5647, DOI: 10.1111/bdi.13041
- ZABOR E C ET AL: "Randomized Controlled Trials", CHEST, vol. 158, no. 1S, 2 July 2020 (2020-07-02), pages S79 - S87, XP093380308, DOI: doi.org/10.1016/j.chest.2020.03.013
- PAVULURI MANI N ET AL: "Double-blind randomized trial of risperidone versus divalproex in pediatric bipolar disorder", BIPOLAR DISORDERS, vol. 12, no. 6, 27 September 2010 (2010-09-27), DK, pages 593 - 605, XP093380309, ISSN: 1398-5647, DOI: 10.1111/j.1399-5618.2010.00850.x
- KOWATCH ROBERT A. ET AL: "Effect Size of Lithium, Divalproex Sodium, and Carbamazepine in Children and Adolescents With Bipolar Disorder", JOURNAL OF THE AMERICAN ACADEMY OF CHILD & ADOLESCENT PSYCHIATRY ELSEVIER, AMSTERDAM, NL, vol. 39, no. 6, 1 June 2000 (2000-06-01), pages 713 - 720, XP085815392, ISSN: 0890-8567, [retrieved on 20091222], DOI: 10.1097/00004583-200006000-00009
- BOWDEN CHARLES L. ET AL: "A Randomized, Placebo-Controlled 12-Month Trial of Divalproex and Lithium in Treatment of Outpatients With Bipolar I Disorder", ARCHIVES OF GENERAL PSYCHIATRY, vol. 57, no. 5, 1 May 2000 (2000-05-01), US, pages 481, XP093380312, ISSN: 0003-990X, DOI: 10.1001/archpsyc.57.5.481
- AHMAD A, SHEIKH S, SHAH T, REDDY MS, PRASAD BSV, VERMA KK, CHANDRAKANT BB, PAITHANKAR M, KALE P, SOLANKI RV, PATEL R, BARKATE H, A: "Endoxifen, a New Treatment Option for Mania: A Double‐Blind, Active‐Controlled Trial Demonstrates the Antimanic Efficacy of Endoxifen", CTS-CLINICAL AND TRANSLATIONAL SCIENCE, vol. 9, no. 5, 1 October 2016 (2016-10-01), pages 252 - 259, XP055855837, ISSN: 1752-8054, DOI: 10.1111/cts.12407

## Description

### FIELD OF THE INVENTION

The present disclosure relates to endoxifen citrate for use in a method for treating bipolar I disorder in a patient, said method consists of: administering to the patient a dose of 8 mg of endoxifen citrate in an enteric coated tablet once per day for at least 21 days; wherein the patient is not administered rescue medication during the treatment; and wherein the rescue medication is selected from the group consisting of: lithium, valproate, lamotrigine, benzodiazepines and anticholinergics.

### BACKGROUND OF THE INVENTION

Bipolar disorder is currently a major health problem. Bipolar disorder is a chronic, debilitating illness that affects up to 3 % of the US population. It causes significant morbidity and imposes a burden on the society. The causes of bipolar disorder are still unknown, and no agent has been specifically developed on the basis of an understanding of the pathophysiology of the illness or mechanism of action for effective treatments.

Several drugs have been approved for treatment of bipolar disorder, such as lithium, valproate or divalproex sodium, carbamazepine, and atypical antipsychotics for the treatment of acute bipolar mania. While these drugs have provided relief for many individuals with bipolar disorder, significant issues with tolerability, efficacy, and attempt suicide or have suicidal behaviour still remain. Further, there is also a need of rescue medications. Divalproex has a good tolerability but a high discontinuation rate. There is also a continuous need to observe the therapeutic dose monitoring in patients during the treatment. The clinicians, for example, may find themselves in situations in which better tolerated agents are less effective, and vice versa. Also, the adherence to the treatment is affected by adverse effects such as sedation, weight gain, thrombocytopenia, and thyroid disorders.

Available treatments help a substantial proportion of patients, but are not beneficial for an estimated 40-50 % of the population.

Ahmad A, Sheikh S, Shah T, Reddy MS, Prasad B, Verma KK, Chandrakant BB, Paithankar M, Kale P, Solanki RV, Patel R, Barkate H, Ahmad I. Endoxifen, a New Treatment Option for Mania: A Double-Blind, Active-Controlled Trial Demonstrates the Antimanic Efficacy of Endoxifen. Clin Transl Sci. 2016 Oct; 9(5): 252-259 discloses the antimanic properties of endoxifen in patients with bipolar I disorder (BPD I) with current manic or mixed episode. In a double-blind, active-controlled study, 84 subjects with BPD I were randomly assigned to receive endoxifen (4 mg/day or 8 mg/day) or divalproex in a 2:1 ratio. Patients orally administered 4 mg/day or 8 mg/day endoxifen showed significant improvement in mania assessed by the Young Mania Rating Scale as early as 4 days.

### SUMMARY OF THE INVENTION

Protein kinase C (PKC) appears to have a role in bipolar disorder. PKC is involved in controlling the function of proteins through the phosphorylation of hydroxyl groups of serine and threonine amino acid residues on these proteins, which are known to play a vital role in cell signalling pathways. It regulates multiple neuronal processes implicated in mood regulation. In current clinical practice, mood stabilizers and antidepressants have been shown to modulate the PKC pathway. Disrupted PKC activity has been found both in post-mortem brains and platelet from patients with mood disorders. Accumulating evidence suggests an imbalance of the PKC signalling system in mood disorders. Thus, PKC is considered as a novel molecular target for the development of innovative medicine for bipolar disorder.

Targeting the PKC signalling pathway for bipolar disorder can improve the patient compliance, when therapeutic dose monitoring is not required in patient, and such treatment can provide significant improvement in mania and depression.

Endoxifen is a non-steroidal selective estrogen receptor modulator (SERM) of the triphenylethylene group. It is an active metabolite of tamoxifen and has been found to be effective in patients that have failed previous hormonal therapies (tamoxifen, aromatase inhibitors, and fulvestrant). The prodrug tamoxifen is metabolized by the CYP2D6 enzyme to produce afimoxifene (4-hydroxytamoxifen) and endoxifen. The chemical name of endoxifen citrate is (Z) - 1 - (4-Hydroxyphenyl) - 1- (4 - [2 -(monomethylamino) ethoxy] phenyl} - 2 - pheny 1 -1-butene citrate. The empirical formula of endoxifen citrate is C₂₅H₂₇NO₂-C₆H₈O₇, and has following chemical structure as given below (formula I):

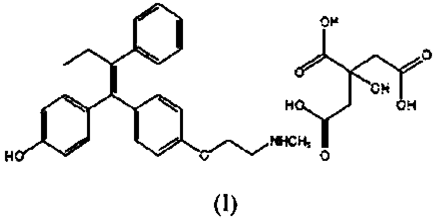

The exact mechanism by which endoxifen exerts its therapeutic effects have not been established in bipolar disorder. However, the efficacy of endoxifen could be mediated through PKC. The PKC represents a family of enzymes highly enriched in brain, where it plays a major role in regulating both pre- and post-synaptic aspects of neurotransmission. Excessive activation of PKC results in symptoms related to bipolar disorder. The PKC signalling pathway is clearly a target for the actions of two structurally dissimilar antimanic agents - lithium and valproate. Tamoxifen, a widely used breast cancer drug is also known to inhibit PKC and demonstrate antimanic properties in human. Endoxifen exhibited four-fold higher potency compared to tamoxifen in inhibiting the PKC activity and is not dependent on the isozyme cytochrome P450 2D6 (CYP2D6) for action on the target tissues. Endxifen is a PKC inhibitor and is effective in the treatment of bipolar disorder. Further, endoxifen has a broad therapeutic index as compared to divalproex sodium.

The present invention relates to endoxifen citrate for use in a method for treating bipolar I disorder in a patient, said method consisting of: administering to the patient a dose of 8 mg of endoxifen citrate in an enteric coated tablet once per day for at least 21 days; wherein the patient is not administered rescue medication during the treatment; and wherein the rescue medication is selected from the group consisting of: lithium, valproate, lamotrigine, benzodiazepines and anticholinergics.

### DETAILED DESCRIPTION OF THE INVENTION

All terms as used herein in this application, unless otherwise stated, shall be understood in their ordinary meaning as known in the art. Other more specific definitions for certain terms as used in the present application are as set forth below and are intended to apply uniformly throughout the specification and claims unless an otherwise expressly set out definition provides a broader definition.

For the purposes of the present application, any ranges given include both the lower and the upper end points of the range. Ranges given should be considered approximate, unless specifically stated.

The term "EOT" refers to end of treatment.

The term "therapeutically effective concentration" refers to a concentration of endoxifen in plasma which is sufficient to decrease or prevent or cure the symptoms associated with a medical condition or infirmity or to normalize body functions in disease or disorders that result in impairment of specific bodily functions.

The term "rescue medication" refers to an additional medication necessary to treat breakthrough or recurring symptoms. For present disclosure, the rescue medications are selected from the group of lithium, valproate, lamotrigine, benzodiazepines, and anticholinergics.

The term "enteric coating" refers to any pharmaceutically acceptable coating preventing the release of the active agent in the stomach and sufficiently disintegrating in the intestine tract (by contact with approximately neutral or alkaline intestine juices) to allow the resorption of the active agent through the walls of the intestinal tract. The enteric coating remains intact in the acidic environment of the stomach and then solubilize in the more alkaline environment of the small intestine. Generally speaking, enteric coating helps in preventing gastric mucosal irritation and can be used for acid labile drugs which gets denatured in acidic medium.

In an embodiment the patient has manic episodes with or without mixed features.

In an embodiment the patient has depression or associated with depressive episodes.

### Clinical study data:

Clinical study of an enteric coated tablet comprising endoxifen citrate formulation was carried out by administering to the patient doses of 8 mg of endoxifen citrate in an enteric coated tablet once per day for at least 21 days according to the present application.

### Design of the study:

The clinical study was a multicenter, randomized, double-blind, double-dummy, active controlled, parallel study to assess the efficacy and safety of endoxifen enteric coated tablet 8 mg and divalproex sodium extended release tablet 1000 mg in patients of bipolar I disorders.

Total 228 patients were enrolled in the study. All 228 patients were qualified for safety trial.

At the EOT, none of the patient required rescue medications, no rescue medications were administered to any of the patients, and none of the patient discontinue from the study.

## Claims

1. Endoxifen citrate for use in a method for treating bipolar I disorder in a patient, said method consisting of:
administering to the patient a dose of 8 mg of endoxifen citrate in an enteric coated tablet once per day for at least 21 days; wherein the patient is not administered rescue medication during the treatment; and wherein the rescue medication is selected from the group consisting of: lithium, valproate, lamotrigine, benzodiazepines and anticholinergics.

2. Endoxifen citrate for use of claim 1, wherein the patient has manic episodes with mixed features.

3. Endoxifen citrate for use of claim 1, wherein the patient has manic episodes without mixed features.

4. Endoxifen citrate for use of claim 1, wherein the patient has depression.

5. Endoxifen citrate for use of claim 1, wherein the patient has depressive episodes.

## Patentansprüche

1. Endoxifencitrat zur Verwendung in einem Verfahren zum Behandeln einer Bipolar-I-Störung bei einem Patienten, wobei das Verfahren aus Folgendem besteht:
Verabreichen einer Dosis von 8 mg Endoxifencitrat in einer magensaftresistent beschichteten Tablette einmal pro Tag an den Patienten für mindestens 21 Tage; wobei dem Patienten während der Behandlung kein Notfallmedikament verabreicht wird; und wobei das Notfallmedikament aus der Gruppe ausgewählt ist, die aus Folgendem besteht: Lithium, Valproat, Lamotrigin, Benzodiazepinen und Anticholinergika.

2. Endoxifencitrat zur Verwendung nach Anspruch 1, wobei der Patient manische Episoden mit gemischten Merkmalen hat.

3. Endoxifencitrat zur Verwendung nach Anspruch 1, wobei der Patient manische Episoden ohne gemischte Merkmale hat.

4. Endoxifencitrat zur Verwendung nach Anspruch 1, wobei der Patient Depressionen hat.

5. Endoxifencitrat zur Verwendung nach Anspruch 1, wobei der Patient depressive Episoden hat.

## Revendications

1. Citrate d'endoxifène destiné à être utilisé dans un procédé de traitement du trouble bipolaire de type I chez un patient, ledit procédé étant constitué de :
l'administration au patient d'une dose de 8 mg de citrate d'endoxifène sous forme de comprimé à enrobage entérique, une fois par jour pendant au moins 21 jours ; dans lequel aucun médicament de secours n'est administré au patient pendant le traitement ; et dans lequel le médicament de secours est choisi dans le groupe constitué de : lithium, valproate, lamotrigine, benzodiazépines et anticholinergiques.

2. Citrate d'endoxifène destiné à être utilisé selon la revendication 1, dans lequel le patient présente des épisodes maniaques avec caractéristiques mixtes.

3. Citrate d'endoxifène destiné à être utilisé selon la revendication 1, dans lequel le patient présente des épisodes maniaques sans caractéristiques mixtes.

4. Citrate d'endoxifène destiné à être utilisé selon la revendication 1, dans lequel le patient présente une dépression.

5. Citrate d'endoxifène destiné à être utilisé selon la revendication 1, dans lequel le patient présente des épisodes dépressifs.
